# EUROPEAN PATENT SPECIFICATION

(11) **EP 2 387 987 B1**
(45) Date of publication and mention of the grant of the patent: **16.04.2014**
(21) Application number: 09838401.9
(22) Date of filing: 24.12.2009
(51) Int. Cl.: A61K 8/41, A61K 8/31, A61K 8/40, A61Q 5/12, A61K 8/34, A61K 8/42, A61Q 5/02

(54) **HAIR COSMETIC**
KOSMETISCHES PRODUKT FÜR DAS HAAR
SOIN COSMÉTIQUE CAPILLAIRE

(30) Priority: 14.01.2009 JP 2009006145
(43) Date of publication of application: 23.11.2011
(73) Proprietor: Shiseido Co., Ltd., Tokyo 104-8010 (JP)
(72) Inventor: YAMAKI Satoshi, Yokohama-shi Kanagawa 224-8558 (JP); SASAKI Tomoko, Yokohama-shi Kanagawa 224-8558 (JP)
(74) Representative: Schön, Christoph
(86) International application number: PCT/JP2009/071422
(87) International publication number: WO 2010/082436

(56) References cited:
- JP-A- 3 264 516
- JP-A- 2000 086 457
- JP-A- 2005 170 941
- JP-A- 2006 096 725
- JP-A- 2006 096 725
- JP-A- 2007 091 615
- JP-A- 2007 254 304
- FRAGRANCE JOURNAL HENSHUBU KOSHOHIN SEIZOGAKU -GIJUTSU TO JISSAI-, 1ST EDITION, 1ST PRINT, FRAGRANCE JOURNAL LTD. 25 August 2001, pages 299 - 300

## Description

### Technical Field

The present invention relates to a hair cosmetic composition and, more particularly, to a hair cosmetic composition suitable for use as a rinse, a conditioner, etc.

### Background Art

Generally, after hair washing (e.g., shampooing), the gloss of the hair tends to be reduced, and fingers do not smoothly run through the hair. To prevent this, a hair-treating agent such as a rinse or a hair conditioner is used.

The hair-treating agent generally contains, as an additive, a quaternary ammonium salt cationic surfactant. Since the quaternary ammonium salt cationic surfactant effectively imparts the hair with softness and anti-static property, the surfactant is incorporated into hair-treating agents as well as into various hair cosmetic compositions.

For example, Patent Document 1 discloses a wash-out-type hair cosmetic composition which contains a quaternary ammonium salt cationic surfactant, 3-methyl-1,3-butanediol, and petrolatum and which imparts the hair with excellent moistness and softness after hair finishing. Patent Document 2 discloses a hair cosmetic composition which contains an ether-type cation surfactant (quaternary ammonium salt) and petrolatum and which softens the hair through an incorporated oily ingredient and imparts the hair with excellent smoothness and moistness. Patent Document 3 discloses a hair cosmetic composition which contains a polysaccharide, a tertiary amine, and a higher alcohol and which favorably styles the hair and imparts the hair with smoothness.

### Prior Art Documents

### Patent Documents

Patent Document 1: Japanese Patent Application Laid-Open (*kokai*) No. 2000-86457
Patent Document 2: Japanese Patent Application Laid-Open (*kokai*) No. 2003-327513
Patent Document 3: Japanese Patent Application Laid-Open (*kokai*) No. 2005-314303.

JP2006096725 and JPJP200791615 disclose hair treatment compositions having excellent moisture retaining properties and comprising hydroxyethylurea, at least one quaternary ammonium salt cationic surfactant, cetostearyl alcohol and liquid paraffin.

### Summary of the Invention

### Problems to be Solved by the Invention

These days, more and more people suffer hair damage from various causes, and demand has arisen for more satisfactory hair-treating agents, including a hair rinse and a conditioner. However, currently, no hair-treating agent which meets user's satisfaction seems to be provided. Specifically, the techniques disclosed in Patent Documents 1 and 2 do not satisfactorily realize hair moistness and hair smoothness of levels which are currently demanded. Also, the technique disclosed in Document 3 causes stickiness and unfavorable tension of the hair due to incorporation of a polysaccharide and does not attain sufficient softness of the hair.

Thus, an object of the present invention is to provide a hair cosmetic composition suitable for use as a rinse, a conditioner, etc., which composition realizes hair finishing conditions more excellent than those attained by the existing hair cosmetic compositions as mentioned above.

### Means for Solving the Problems

The present inventors have found that the aforementioned object can be attained by a hair cosmetic composition containing a specific hydroxyalkylurea and petrolatum.

Accordingly, the present invention provides a hair cosmetic composition selected from the group consisting of hair rinse, hair conditioner, hair treatment, hair shampoo and rinse-in shampoo, comprising the following ingredients (1) to (5) :
(1) petrolatum in an amount of 0.05 to 5 mass% with respect to the cosmetic composition;
(2) N-(2-hydroxyethyl)urea in an amount of 0.01 to 20 mass% with respect to the cosmetic composition;
(3) a tertiary amine cationic surfactant and/or a quaternary ammonium salt cationic surfactant in an amount of 0.2 to 5 mass% with respect to the cosmetic composition;
(4) one or more higher alcohols selected from the group consisting of lauryl alcohol, myristyl alcohol, cetyl alcohol (cetanol), cetostearyl alcohol, stearyl alcohol, arachyl alcohol, behenyl alcohol, oleyl alcohol, jojoba alcohol, chimyl alcohol, and batyl alcohol, in an amount of 0.1 to 20 mass% with respect to the cosmetic composition; and
(5) water. (hereinafter the composition may be referred to as the hair cosmetic composition of the present invention).

The present invention enables provision of a hair cosmetic composition which can finish the hair, after shampooing, with excellent smoothness, suppleness, and moistness and which is suitable for use as a hair-treating agent such as a rinse or a conditioner.

### Modes for Carrying out the Invention

The hair cosmetic composition of the present invention contains, as essential ingredients, the aforementioned (1) to (5).

### Petrolatum

The petrolatum which can be incorporated into the hair cosmetic composition of the present invention is an amorphous hydrocarbon oil mainly including C24 to C34 hydrocarbons, which oil is produced by purifying an ointment-like substance obtained through solvent dewaxing of a vacuum distillation residue of crude oil. The petrolatum may be a commercial purified product generally employed in cosmetic compositions.

The hair cosmetic composition of the present invention has a petrolatum content (with respect to the total amount of cosmetic composition) of 0.05 to 5 mass%. When the petrolatum content is less than 0.01 mass%, enhancement in hair finish effect by virtue of incorporation of petrolatum is likely to be reduced, whereas when the petrolatum content is in excess of 20 mass%, no substantial effect commensurate with addition in large amount is attained, and sensation of the composition in use may be impaired.

The N-(2-hydroxyethyl)urea may be produced through a method generally known in the art, for example, through reaction of a lower alcohol with urea at 120°C for several hours and deammoniation by contact with nitrogen. Alternatively, a commercial product thereof; e.g., a commercial product of N-(2-hydroxyethyl)urea HYDROVANCE (B) (product of Nippon NSC)-may be employed.

The hair cosmetic composition of the present invention has a N-(2-hydroxyethyl)urea content (with respect to the total amount of cosmetic composition) of 0.01 to 20 mass%. When the N-(2-hydroxyethyl)urea content is less than 0.001 mass%, enhancement in hair finish effect by virtue of incorporation of petrolatum is likely to be reduced, whereas when the N-(2-hydroxyethyl)urea content is in excess of 30 mass%, no substantial effect commensurate with addition in large amount is attained.

Tertiary amine cationic surfactant and quaternary ammonium salt cationic surfactant

In the present invention, the tertiary amine cationic surfactant and the quaternary ammonium salt cationic surfactant may be selected from surfactants generally employed in rinse formulations.

Examples of the tertiary amine cationic surfactant include tertiary amine compounds represented by the hollowing formula (I) : 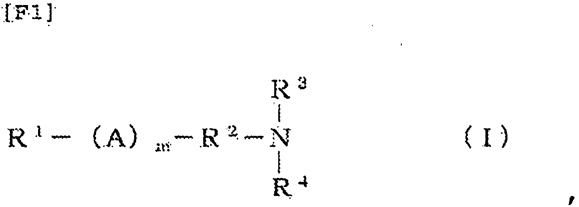 wherein R¹ represents a C6 to C24 linear or branched alkyl or alkenyl group; A represents an ester group, an amide group, or an ether group; m is 0 or 1; R² represents a C1 to C6 linear or branched alkylene or alkylenyl group; and R³ and R⁴, which are identical to or different from each other, each represent a hydrogen atom or a C1 to C6 linear or branched alkyl group.

Examples of the C6 to C24 linear or branched alkyl or alkenyl group of R¹ include hexyl, heptyl, octyl, nonyl, decyl, undecyl, dodecyl, tridecyl, tetradecyl, pentadecyl, hexadecyl, heptadecyl, octadecyl, nonadecyl, icosyl, henicosyl, docosyl, tricosyl, tetracosyl,myristoleyl, palmitoleyl, oleyl, linolyl, linoleyl, ricinoleyl, and isostearyl. Examples of the C1 to C6 linear or branched alkylene or alkylenyl group of R² include methylene, ethylene, trimethylene, tetramethylene, pentamethylene, and hexamethylene. R³ and R⁴, which are identical to or different from each other, each represent a hydrogen atom or a C1 to C6 linear or branchedalkyl group, typically methyl.

Examples of the tertiary amine cationic surfactant also include alkyldimethylammonium salts and alkylbenzyldimethylammonium salts. The alkyl groups forming the tertiary ammonium salt are, for example, about C16 to about C35 linear or branched alkyl groups. Typical examples thereof include linear alkyl groups such as hexadecyl, heptadecyl, octadecyl, nonadecyl, icosyl, henicosyl, docosyl, tricosyl, tetracosyl, pentacosyl, hexacosyl, heptacosyl, octacosyl, nonacosyl, triacontyl, hentriacontyl, dotriacontyl, tritriaconryl, tetratriacontyl, and pentatriacontyl. Among them, hexadecyl (C16), octadecyl (C18), docosyl (C22), etc. are preferred linear alkyl groups. Examples of the counter anion forming the ammonium salts include halide ions such as chloride, iodide, and bromide; and organic anions such as methosulfate, ethosulfate, methophosphate, and ethophosphate. The counter anion is typically chloride.

Examples of the quaternary ammonium salt cationic surfactant include alkyltrimethyl ammonium salts. The alkyl groups and anions forming the quaternary ammonium salt cationic surfactant are the same as described in relation to the aforementioned tertiary ammonium salt.

The tertiary amine cationic surfactants or the quaternary ammonium salt cationic surfactants may be used singly or in combination of two or more species. The tertiary amine cationic surfactant and the quaternary ammonium salt cationic surfactant may be used in combination.

The total amount of These cationic surfactants is 0.2 to 5 mass% with respect to the total amount of the cosmetic composition. When the total amount of cationic surfactants is less than 0.1 mass%, essential functions of the hair cosmetic composition of the present invention are difficult to attain, whereas when the total amount is in excess of 10 mass%, improvement in performance of the cosmetic product commensurate with addition is difficult to attain.

### Water

Water employable in the cosmetic composition may be any of ion-exchanged water, purified water, tap water, and natural water, and the type and amount of water may be selected in accordance with the form and mode of use of the hair cosmetic composition of the present invention. Specifically, in a typical manner, the cosmetic composition is formed from the aforementioned essential ingredients (mass%), other ingredients selected in consideration of the form and mode of use of the hair cosmetic composition (mass%), and water as balance.

### Higher alcohols

Through incorporation of a higher alcohol into the hair cosmetic composition of the present invention, the hair cosmetic composition particularly suitably serves as a hair-treating agent such as a rinse or a hair conditioner. In other words, when these ingredients, the aforementioned tertiary amine cationic surfactant and/or quaternary ammonium salt cationic surfactant, and water come into contact with one another, a gel having a layer structure (lamella structure) is formed. Thus, through addition of oily ingredients such as the aforementioned petrolatum to this gel system to thereby form an emulsion, a gel in which oil droplets are dispersed is produced. This product is suitable for a rinse or a conditioner in the form of milky lotion or cream.

The higher alcohols are selected from the group consisting of lauryl alcohol, myristyl alcohol, cetyl alcohol (cetanol), cetostearyl alcohol, stearyl alcohol, arachyl alcohol, behenyl alcohol, oleyl alcohol, jojoba alcohol, chimyl alcohol, and batyl alcohol.

The hair cosmetic composition of the present invention has a higher alcohol content of 0.1 to 20 mass% with respect to the total amount of the cosmetic composition.

If required, the hair cosmetic composition of the present invention may further contain a certain additional ingredient other than the aforementioned ingredients in such an amount that the effects of the present invention are not substantially impaired. For example, in the case where the hair cosmetic composition of the present invention is a shampoo, typical examples of the additional ingredient employed include anionic surfactants such as alkyl sulfate salts, polyoxyethylene alkyl ether sulfate salts, acylmethyltauric acid, and N-acylglutamic acid salts; amphoteric surfactants such as alkylbetaine, alkylamidobetaine, and imidazoliniumbetaine; and nonionic surfactants such as fatty acid alkanolamines.

Regardless of the form, the hair cosmetic composition may further contain other additives. Specific examples include oily ingredients (other than petrolatum and higher alcohols) such as higher fatty acias, hydrocarbon oils, ester oils, and silicone oils (e.g., amino-modified silicones and methylpolysiloxanes); humectants such as glycerin, propylene glycol, 1,3-butylene glycol, and polyethylene glycol; conditioning agents such as cationic polymer (e.g., cationized cellulose); anti-dandruff agents such as trichlolorocarbanilide, sulfur, zinc pyrithione, and isopropylmethyl phenol; a thickener; a viscosity-controlling agent; an emulsifying agent; a sequestering agent; a UV-absorber; an anti-oxidant; an antiseptic agent; powder ingredients; hair-growing agents such as a blood flow-promoting agent, a local stimulant, a hair follicle-activating agent, an anti-androgenic agent, an antiseborrheic agent, a keratoolytic agent, a bactericide, an anti-inflammatory agent, amino acid, vitamins, and a crude drug extract; a pH-regulating agent, a dye; perfume; and lower alcohols.

### <The hair cosmetic composition of the present invention

The hair cosmetic composition of the present invention may be produced through a suitable method selected in accordance with the form and product type of the composition. In one typical method, the aforementioned essential ingredients and optional ingredients are dissolved in water. Examples of the product type include hair rinse, hair conditioner, hair treatment, hair shampoo, and rinse-in-shampoo

### Examples

The present invention will next be described in detail by way of examples, which should not be construed as limiting the invention thereto. Unless otherwise specified, the amount of each ingredient is on the basis of mass%.

### <method of evaluation>

The hair cosmetic composition of the present invention was evaluated through the following methods.

### (1) Smoothness, suppleness, and moistness of the hair after finishing

Six male panelists were shampooed with an ordinary shampoo (having no rinsing effect), and the remaining shampoo was sufficiently washed, off. Then, each panelist uniformly applied a test cosmetic composition sample once by the palms to the hair in a wet state, and the thus-treated hair was thoroughly rinsed off. Thereafter, the hair was dried with a towel and by means of a drier. The conditions of the dried hair were evaluated by the panelist in terms of smoothness, suppleness, and moistness, based on the following ratings.
○○ (excellent) ; All the panelists (6 panelists) evaluated the hair conditions as smooth, supple, or moist.
○ (good): Five of the six panelists evaluated the hair conditions as smooth, supple, or moist.
○Δ (fairly good): Four of the six panelists evaluated the hair conditions as smooth, supple, or moist.
Δ (fair): Three of the six panelists evaluated the hair conditions as smooth, supple, or moist.
ΔX (somewhat poor): Two of the six panelists evaluated the hair conditions as smooth, supple, or moist.
X (poor): None or one of the six panelists evaluated the hair conditions as smooth, supple, or moist.

### (2) Bending stress (B value: gf.cm²/cm)

The bending stress of the hair is a parameter which indicates the hardness, tension, or toughness of the hair. In this test, the bending stress of a commercial human tress was measured. More specifically, the tress was shampooed, a test cosmetic composition sample was applied to the tress, and the tress was rinsed off, in a manner similar to the aforementioned test, followed by drying by means of a drier. The bending stress of the thus-treated tress was determined by means of a hair bending tester KES-SH (product of Kato Tech Co., Ltd.). The smaller the bending stress, the softer the hair.

### (3) Average friction coefficient

The average friction coefficient of the hair is a parameter which indicates the smoothness of the hair. In this test, the friction coefficient of a commercial human tress was measured. More specifically, the tress was shampooed, a test cosmetic composition sample was applied to the tress, and the tress was rinsed off, in a manner similar to the aforementioned test, followed by drying by means of a drier. The average friction coefficient of the thus-treated tress was determined by means of an organoleptic roughness/friction tester KES-SE-U (product of Kato Tech Co., Ltd.). The smaller the average friction coefficient, the smoother the hair surface.

### Test Examples

### (1) Evaluation of actually used cosmetic composition

Ingredients shown in Table 1 were mixed at proportions shown in Table 1 and dissolved under stirring at 80°C. The solution was cooled, to thereby yield rinse products. Each rinse product was evaluated in terms of smoothness, suppleness, and moistness, through the same procedure as described above. The results are also shown in Table 1.

**[Table 1]**

| | Comp. Ex. 1 | Comp. Ex. 2 | Comp. Ex. 3 | Ex. 1 | Comp. Ex. 4 | Comp. Ex. 5 | Ex. 2 | Comp. Ex. 6 | Comp. Ex. 7 | Ex. 3 |
|---|---|---|---|---|---|---|---|---|---|---|
| Behentrimonium chloride | --- | 2 | 2 | 2 | --- | --- | --- | --- | --- | --- |
| Stearyl trimethylammonium chloride | --- | --- | --- | --- | 2 | 2 | 2 | --- | --- | --- |
| Dimethylaminopropylstearamide | --- | --- | --- | --- | --- | --- | --- | 2 | 2 | 2 |
| Cetanol | --- | 4 | --- | 4 | 4 | --- | 4 | 4 | --- | 4 |
| Hydroxyethylurea | --- | 2 | --- | 2 | 2 | --- | 2 | 2 | --- | 2 |
| Petrolatum | --- | --- | 2 | 2 | --- | 2 | 2 | --- | 2 | 2 |
| Dipropylene glycol | --- | 8 | 8 | 8 | 8 | 8 | 8 | 8 | 8 | 8 |
| Phenoxyethanol | --- | 0.6 | 0.6 | 0.6 | 0.6 | 0.6 | 0.6 | 0.6 | 0.6 | 0.6 |
| Perfume | --- | q.s. | q.s. | q.s. | q.s. | q.s. | q.s. | q.s. | q.s. | q.s. |
| Purified water | 100 | balance | balance | balance | balance | balance | balance | balance | balance | balance |
| Smoothness | Δ | ○ | ○Δ | ○○ | ○Δ | ○○ | ○○ | ○ | ○Δ | ○○ |
| Suppleness | Δ | ○Δ | ○Δ | ○ | ○Δ | ○Δ | ○ | ○ | ○Δ | ○○ |
| Moistness | Δ | ○Δ | ○ | ○○ | ○Δ | ○ | ○○ | ○ | ○○ | ○○ |

As is clear from Table 1, in the case where dimethylaminopropylstearamide (tertiary amine compound) was employed, or in the case where behentrimonium chloride or stearyl trimethylammonium chloride (quaternary ammonium salt cationic surfactant) was employed, satisfactory smoothness, suppleness, and moistness were observed in the hair through incorporation of petrolatum and hydroxyethylurea (N-(2-hydroxyethyl) urea, HYDROVANCE (B) (product of Nippon NSC), hereinafter the same product was employed) in combination into the hair cosmetic composition (Examples 1 to 3). In contrast, touch feeling of the hair after treatment with a composition not containing petrolatum or hydroxyethylurea (Comparative Examples 2 to 7) was inferior to that attained in the Examples.

### (2) Evaluation through bending stress test

Rinse products having formulations shown in Table 2 were subjected to a bending test in the aforementioned manner. Table 2 also shows the results.

**[Table 2]**

| | Comp. Ex. 1 | Comp. Ex. 8 | Ex. 4 | Comp. Ex. 9 | Ex. 5 | Comp. Ex. 10 | Ex. 6 |
|---|---|---|---|---|---|---|---|
| Behentrimonium chloride | --- | 2 | 2 | --- | --- | --- | --- |
| Stearyl trimethylammonium chloride | --- | --- | --- | 2 | 2 | --- | --- |
| Dimethylaminopropylstearamide | --- | --- | --- | --- | --- | 2 | 2 |
| Cetanol | --- | --- | 4 | --- | 4 | --- | 4 |
| Hydroxyethylurea | --- | --- | 2.5 | --- | 2.5 | --- | 2.5 |
| Petrolatum | --- | 3 | 3 | 3 | 3 | 3 | 3 |
| Propylene glycol | --- | 8 | 8 | 8 | 8 | 8 | 8 |
| Sorbitol | --- | 10 | 10 | 10 | 10 | 10 | 10 |
| PEG (average molecular weight: 20,000) | --- | 0.1 | 0.1 | 0.1 | 0.1 | 0.1 | 0.1 |
| Phenoxyethanol | --- | 0.6 | 0.6 | 0.6 | 0.6 | 0.6 | 0.6 |
| Aminopropyldimethicone | --- | 1 | 1 | 1 | 1 | 1 | 1 |
| Dimethicone | --- | 3 | 3 | 3 | 3 | 3 | 3 |
| Perfume | --- | q.s. | q.s. | q.s. | q.s. | q.s. | q.s. |
| Purified water | 100 | balance | balance | balance | balance | balance | balance |
| Before bending stress treatment | 0.6222 | 0.7571 | 0.8303 | 0.8369 | 0.848 | 0.7727 | 0.7889 |
| After bending stress treatment | 0.6223 | 0.671 | 0.732 | 0.7849 | 0.793 | 0.7605 | 0.7645 |
| Percentage of bending stress change | 1.000161 | 0.886277 | 0.881609 | 0.937866 | 0.935142 | 0.984211 | 0.969071 |

In Table 2, PEG refers to polyethylene glycol. The same is applied throughout the specification.

As is clear from Table 2, through incorporation of petrolatum, hydroxyethylurea, and cetanol (higher alcohol) in combination into the cosmetic composition containing a tertiary amine compound or a quaternary amine cationic surfactant, the bending stress of the hair decreased, and the smoothness and moistness of the hair were enhanced (Example 4 vs. Comparative Example 8, Example 5 vs. Comparative Example 9, and Example 6 vs. Comparative Example 10)

### (3) Evaluation in terms of average friction coefficient

Rinse products having formulations shown in Table 3 were subjected to a friction test in the aforementioned manner. Table 3 also shows the results.

**[Table 3]**

| | Comp. Ex. 11 | Comp. Ex. 12 | Ex. 7 |
|---|---|---|---|
| Dimethylaminopropylstearamide | 2 | 2 | 2 |
| Stearyl alcohol | 5 | --- | 5 |
| Mineral oil | 1 | 1 | 1 |
| Hydroxyethylurea | 1.2 | --- | 1.2 |
| Petrolatum | --- | 3 | 3 |
| Water | balance | balance | balance |
| Glycerin | 4 | 4 | 4 |
| PEG (average molecular weight: ≥20,000) | 0.1 | 0.1 | 0.1 |
| Phenoxyethanol | 0.7 | 0.7 | 0.7 |
| Aminopropyldimethicone | 2 | 2 | 2 |
| Dimethicone | 1 | 1 | 1 |
| Perfume | q.s. | q.s. | q.s. |
| Purified water | balance | balance | balance |
| Av. friction coefficient | 0.428 | 0.409 | 0.392 |

As is clear from Table 3, the rinse product of the present invention containing petrolatum and hydroxyethylurea in combination (Example 7) realized a small average friction coefficient and a smooth hair surface, as compared with comparative rinse products (Comparative Examples 11 and 12) containing no petrolatum or hydroxyethylurea.

Formulation Examples of the present invention will be described. These Formulation Examples were prepared through the same method as that of the rinse products in Table 1.

### Formulation Example 1: Hair rinse

| Ingredients | Amount (mass%) |
|---|---|
| Dimethylaminopropylstearamide | 2.5 |
| Petrolatum | 1.2 |
| N-(2-hydroxyethyl)urea | 3.5 |
| Stearyl alcohol | 5.0 |
| Dipropylene glycol | 5.0 |
| Glutamic acid | 0.7 |
| Mineral oil | 2.0 |
| Phenoxyethanol | 0.8 |
| Perfume | q.s. |
| Purified water | balance |

### Formulation Example 2: Hair treatment

| Ingredients | Amount (mass%) |
|---|---|
| Dimethylaminopropylstearamide | 0.5 |
| Petrolatum | 0.5 |
| Behentrimonium chloride | 1.4 |
| Cetanol | 0.8 |
| Stearyl alcohol | 4.0 |
| N-(2-hydroxyethyl) urea | 1.1 |
| Aminopropyldimethicone (av, polymerisation degree: 5,000) | 3.0 |
| Glycerin | 5.0 |
| Propylene glycol | 10.0 |
| Phenoxyethanol | 0.6 |
| Perfume | q.s. |
| Purified water | balance |

### Formulation Example 3: After hair color treatment

| Ingredients | Amount (mass%) |
|---|---|
| Stearyl trimethylammonium chloride | 3.2. |
| Petrolatum | 4.0 |
| N-(2-hydroxyethyl)urea | 0.8 |
| Dimethicone (av. polymerisation degree: 7,000) | 5.0 |
| Behenyl alcohol | 4.5 |
| Dipropylene glycol | 8.0 |
| Sorbitol | 10.0 |
| Phenoxyethanol | 0.7 |
| Dimethyldiallyl ammonium chloride-acrylamide copolymer liquid | 0.3 |
| Perfume | q.s. |
| Purified water | balance |

## Claims

1. A hair cosmetic composition selected from the group consisting of hair rinse, hair conditioner, hair treatment, hair shampoo, and rinse-in-shampoo, comprising the following ingredients (1) to (5) :
(1) petrolatum in an amount of 0.05 to 5 mass% with respect to the cosmetic composition;
(2) N- (2-hydroxyethyl) urea in an amount of 0.01 to 20 mass% with respect to the cosmetic composition;
(3) tertiary amine cationic surfactant and/or a quaternary ammonium salt cationic surfactant in an amount of 0.2 to 5 mass% with respect to the cosmetic composition;
(4) one or more higher alcohols selected from the group consisting of lauryl alcohol, myristyl alcohol, cetyl alcohol (cetanol), cetostearyl alcohol, stearyl alcohol, arachyl alcohol, behenyl alcohol, oleyl alcohol, jojoba alcohol, chimyl alcohol, and batyl alcohol, in an amount of 0.1 to 20 mass% with respect to the cosmetic composition; and
(5) water.

2. The hair cosmetic composition according to claim 1, which composition is hair rinse or hair treatment.

## Patentansprüche

1. Haarkosmetikzusammensetzung, die aus der Gruppe ausgewählt ist, die aus einer Haarspülung, einem Haarconditioner, einer Haarbehandlung, einem Haarshampoo und einem Rinse-in-Shampoo besteht, wobei die Haarkosmetikzusammensetzung die folgenden Bestandteile (1) bis (5) umfasst:
(1) Rohvaseline in einer Menge von 0,05 bis 5 Massenprozent, bezogen auf die Kosmetikzusammensetzung;
(2) N-(2-Hydroxyethyl)harnstoff in einer Menge von 0,01 bis 20 Massenprozent, bezogen auf die Kosmetikzusammensetzung;
(3) kationisches grenzflächenaktives Mittel in Form eines tertiären Amins und/oder kationisches grenzflächenaktives Mittel in Form eines quaternären Ammoniumsalzes in einer Menge von 0,2 bis 5 Massenprozent, bezogen auf die Kosmetikzusammensetzung;
(4) ein oder mehrere höhere Alkohole, die aus der Gruppe ausgewählt sind, die aus Laurylalkohol, Myristylalkohol, Cetylalkohol (Cetanol), Cetostearylalkohol, Stearylalkohol, Arachylalkohol, Behenylalkohol, Oleylalkohol, Jojobaalkohol, Chimylalkohol und Batylalkohol besteht, in einer Menge von 0,1 bis 20 Massenprozent, bezogen auf die Kosmetikzusammensetzung; und
(5) Wasser.

2. Haarkosmetikzusammensetzung nach Anspruch 1, wobei die Zusammensetzung eine Haarspülung oder eine Haarbehandlung ist.

## Revendications

1. Composition de soin cosmétique capillaire sélectionnée parmi le groupe constitué d'un produit de rinçage capillaire, d'un après-shampoing, d'un traitement capillaire, d'un shampoing capillaire, d'un shampoing deux-en-un, comprenant les ingrédients suivants (1) à (5) :
(1) du pétrolatum dans une quantité de 0,05 à 5 % en masse par rapport à la composition de soin cosmétique ;
(2) de la N-(2-hydroxyéthyl)urée dans une quantité de 0,01 à 20 % en masse par rapport à la composition de soin cosmétique ;
(3) un tensioactif cationique à base d'amine tertiaire et/ou un tensioactif cationique à base de sel d'ammonium quaternaire dans une quantité de 0,2 à 5 % en masse par rapport à la composition de soin cosmétique ;
(4) un ou plusieurs alcools supérieurs sélectionnés parmi le groupe constitué d'alcool laurylique, d'alcool myristylique, d'alcool cétylique (cétanol), d'alcool cétostéarylique, d'alcool stéarylique, d'alcool arachylique, d'alcool béhénylique, d'alcool oléylique, d'alcool de jojoba, d'alcool chimylique, et d'alcool batylique, dans une quantité de 0,1 à 20 % en masse par rapport à la composition de soin cosmétique ; et
(5) de l'eau.

2. Composition de soin cosmétique capillaire selon la revendication 1, laquelle composition est un produit de rinçage capillaire ou un traitement capillaire.
